# EUROPÄISCHE PATENTSCHRIFT

(11) **EP 1 796 541 B1**
(45) Veröffentlichungstag und Bekanntmachung des Hinweises auf die Patenterteilung: **17.06.2009**
(21) Anmeldenummer: 05787151.9
(22) Anmeldetag: 05.09.2005
(51) Int. Cl.: A61B 6/04

(54) **MAMMOGRAPHIEGERÄT MIT HÖHENVERSTELLBAREM OBJEKTTISCH**
MAMMOGRAPHY DEVICE HAVING A HEIGHT-ADJUSTABLE OBJECT PLATFORM
APPAREIL POUR MAMMOGRAPHIE A TABLE PORTE-OBJET REGLABLE EN HAUTEUR

(30) Priorität: 09.09.2004 DE 102004043739
(43) Veröffentlichungstag der Anmeldung: 20.06.2007
(73) Patentinhaber: SIEMENS AKTIENGESELLSCHAFT, 80333 München (DE)
(72) Erfinder: RAMSAUER, Martin, 90602 Pyrbaum (DE); TICHY, Peter, 91080 Uttenreuth (DE)
(86) Internationale Anmeldenummer: PCT/EP2005/054346
(87) Internationale Veröffentlichungsnummer: WO 2006/027341

(56) Entgegenhaltungen:
- EP-A- 0 406 455
- GB-A- 2 212 040
- GB-A- 2 331 360
- US-A- 4 901 335
- US-A- 5 068 887

## Beschreibung

Die Erfindung betrifft ein Mammographiegerät mit höhenverstellbarem Objekttisch gemäß Patentanspruch 1 bzw, gemäß Patentanspruch 3.

Mit Mammographie bezeichnet man eine Standardmethode der Diagnostik, insbesondere zur vorbeugenden Untersuchung auf Brustkrebs. Mammographiegeräte sind so konstruiert, dass die zu untersuchende Brust vor der Röntgenaufnahme auf einem Objekttisch dieses Gerätes zu platzieren ist; dazu ist es zunächst notwendig, den Objekttisch auf die Brusthöhe der Patientin einzustellen. Diese Höheneinstellung wird vorgenommen, während die Patientin vor dem Mammographiegerät steht. Bei den gebräuchlichen Mammographiegeräten ist diese Höhenjustage mit dem bloßen Auge abzuschätzen, was oftmals erst nach mehreren Korrekturen gelingt. Solche Mammographiegeräte sind beispielsweise jeweils aus der EP 0 406 455 A1 und der US 4,901,335 bekannt.

Der Erfindung liegt die Aufgabe zugrunde, ein Mammographiegerät zu schaffen, das mit einfachen Mitteln eine genaue und schnelle Höhenanpassung des Objekttisches an den Untersuchungsbereich der Patientin ermöglicht.

Die Lösung dieser Aufgabe gelingt durch ein Mammographiegerät gemäß Patentanspruch 1 bzw. gemäß Patentanspruch 3; vorteilhafte Ausgestaltungen sind jeweils Gegenstand der Unteransprüche.

Die gemäß Patentanspruch 1 vorgesehene erfindungsgemäße Projektion eines von der jeweiligen Höhe des Objekttisches abhängigen optischen Signals auf die Patientin erlaubt, insbesondere bei einer entsprechend einer Ausgestaltung der Erfindung vorgesehenen Abbildung der Höhe des Objekttisches auf den Körper der Patientin, durch die im Vergleich mit der Augenabschätzung bessere Anpeilmöglichkeit die exakte Höhenanpassung des Objekttisches bei beschleunigtem Arbeitsablauf, so dass für die Bedienperson des Mammographiegerätes mühsame und für die Patientin belastende mehrfache Nachjustagen der Objekttischhöhe vermeidbar sind.

Besonders einfach lässt sich die Projektion durch die Verwendung eines Lichtstrahls als optisches Signal erreichen. Eine sehr scharfe und daher besonders gut erkennbare Projektion ergibt sich bei Verwendung eines Laserstrahls.

Die gemäß Patentanspruch 3 vorgesehene erfindungsgemäße zusätzliche anschließende Reflexion des optischen Signals von der Patientin auf das Mammographiegerät erlaubt, insbesondere bei nach einer Ausgestaltung vorgesehener Verwendung eines für das optische Signal empfindsamen Sensors, eine besonders einfache und zuverlässige Überwachung; konstruktiv kompakt und einfach ist der Sensor mit dem Objekttisch zusammengefasst. Zur weiteren Vereinfachung ist nach einer Ausgestaltung eine selbsttätige Höheneinstellung des Objekttisches in Abhängigkeit des von dem Sensor gemessenen, reflektierten Signals vorgesehen.

Durch die DE 103 05 640 A1 ist eine Mammographie-Vorrichtung mit einem Ultraschallmessfühler und mit einem Positionssensor des Ultraschallmessfühlers bekannt, wobei als Positionssensor insbesondere ein Fernsensor zur kontaktlosen Positionsbestimmung des Ultraschallmessfühlers mittels eines optischen Signals vorgesehen ist; diese Ausbildung einer Mammographie-Vorrichtung ist auf die Aufgabe gerichtet, die Durchführung unterschiedlicher Röntgen- und Ultraschallbelichtungen zu ermöglichen, ohne dadurch den Patienten zu bewegen und die Vorrichtung neu anzupassen. Das Wissen über die mit dem Positionssensor ermittelte Position des Messfühlers ermöglicht insbesondere die Verarbeitung eines mit dem Ultraschallmessfühler erfassbaren Echogramms in Übereinstimmung mit einem mit der Mammographie-Vorrichtung erstellbaren Röntgenbild.

Die Erfindung sowie weitere vorteilhafte Ausgestaltungen der Erfindung gemäß Merkmalen der Unteransprüche werden im folgenden anhand von schematisch dargestellten Ausführungsbeispielen in der Zeichnung näher erläutert, ohne dass dadurch eine Beschränkung der Erfindung auf dieses Ausführungsbeispiel erfolgt; es zeigen:
- FIG 1: in seitlicher Ansicht einen Objekttisch eines Mammographiegerätes mit einem Sender für das optische Signal sowie eine vor dem Objekttisch stehende Patientin vor einer Höheneinstellung des Objekttisches;
- FIG 2: die Anordnung gemäß FIG 1 nach erfolgter Höheneinstellung des Objekttisches;
- FIG 3: in seitlicher Ansicht einen Objekttisch eines Mammographiegerätes mit einem Sender für das optische Signal sowie eine vor dem Objekttisch stehende und mit einem Reflektor zur Reflexion des optischen Signals versehene Patientin vor einer Höheneinstellung des Objekttisches;
- FIG 4: die Anordnung gemäß FIG 3 nach erfolgter Höheneinstellung des Objekttisches.

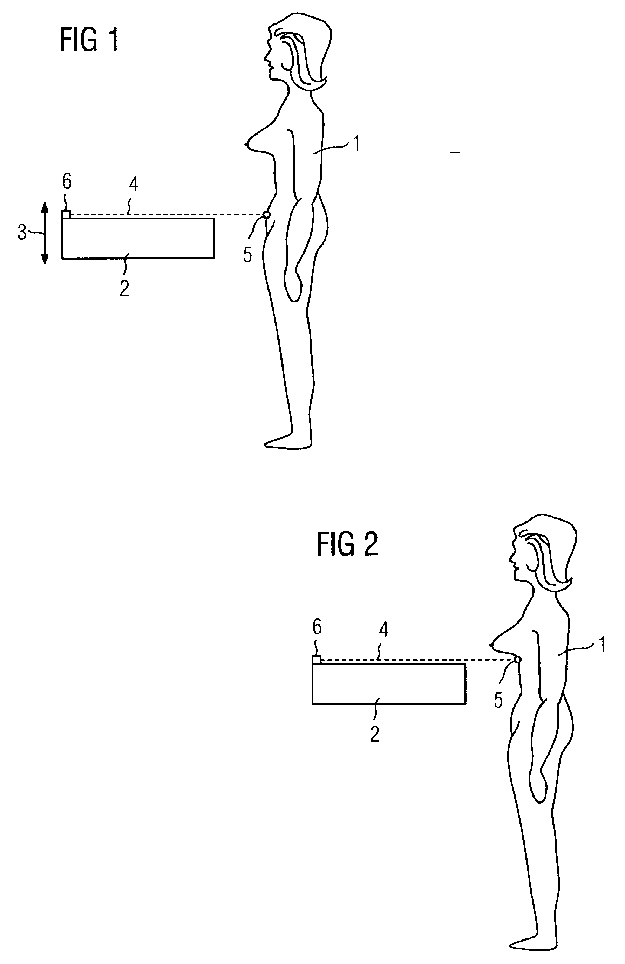

Figur 1 zeigt in seitlicher Ansicht eine Patientin 1 vor einem Objekttisch 2 stehend, dessen Höhe in der durch einen Pfeil 3 angedeuteten Richtung in Bezug auf den Untersuchungsbereich der Patientin 1 einstellbar ist. Zur Höheneinstellung ist ein der jeweiligen Höhe des Objekttisches 2 entsprechendes optisches Signal 4 auf die Patientin 1 projizierbar. Durch die Projektion 5 des optischen Signals 4 ist die Höhe des Objekttisches 2 auf dem Körper der Patientin 1 abbildbar, so dass auf besonders einfache Weise der Objekttisch 2 auf die in Figur 2 dargestellte, für die zur Untersuchung geeignete Höhe einstellbar ist. Figur 1 bzw. Figur 2 zeigt jeweils ein Ausführungsbeispiel, bei dem das optische Signal 4 von einem Sender 6 erzeugt wird, der sich vorteilhaft in baulicher Einheit mit dem Objekttisch 2 befindet.

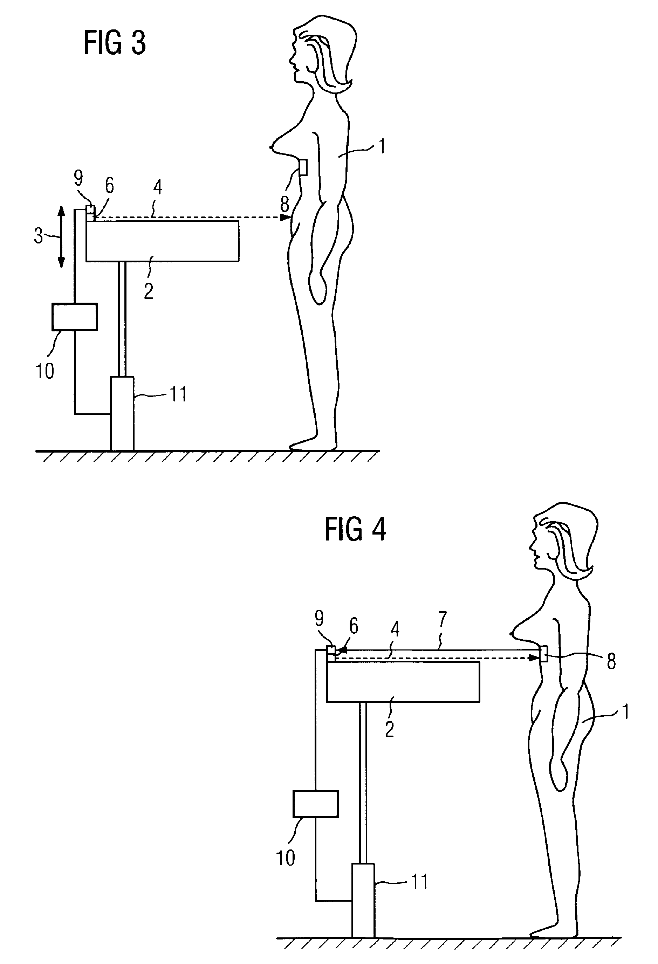

Die Figuren 3 und 4 zeigen eine weitere erfindungsgemäße Lösung, bei der in Weiterführung der Höheneinstellung gemäß der Figuren 1 und 2 das projizierte optische Signal 4 von der Patientin 1 reflektiert wird, wenn der Objekttisch 2 bei seiner Höhenverstellung seine dem Untersuchungsbereich der Patientin 1 entsprechende Höhe erreicht hat; zur Reflexion ist nach einer Ausgestaltung der Erfindung unmittelbar unterhalb des Untersuchungsbereichs ein Reflektor 8 vorgesehen.

Im Einzelnen zeigt Figur 3 zunächst das von einem vorteilhaft mit dem Objekttisch 2 baulich zusammengefassten Sender 6 in Richtung auf die Patientin 1 projizierte optische Signal 4, das die Patientin 1 unterhalb des Reflektors 8 trifft und somit nicht zum Objekttisch 2 reflektiert wird. Die Höhe des Objekttisches 2 ist in Richtung des in Figur 3 dargestellten Pfeils 3 einstellbar. Bei Erreichen der für die Untersuchung passenden Höheneinstellung des Objekttisches 2 trifft das projizierte optische Signal 4 - wie in Figur 4 angedeutet - auf den Reflektor 8 und wird von diesem reflektiert, wobei das reflektierte optische Signal 7 für die Bedienperson des Mammographiegerätes erkennbar ist.

Neben einer Sichterfassung des reflektierten optischen Signals 7 ist nach einer Ausgestaltung der Erfindung ein Sensor 9, insbesondere in baulicher Einheit mit dem Objekttisch 2, vorgesehen, der das reflektierte optische Signal 7 durch das Auslösen eines optischen und/oder akustischen Signals anzeigt. Der Einsatz des Sensors 9 erlaubt außerdem eine Signalerfassung bei Verwendung eines nicht-sichtbaren optischen Signals 4 bzw. 7, wie z.B. Infrarotlicht.

Nach einer weiteren Ausgestaltung der Erfindung dient das von dem Sensor 9 gemessene reflektierte Signal 7 als Kriterium für eine Steuereinheit 10, welche die Höheneinstellung des Objekttisches 2 über einen Höhenantrieb 11 selbsttätig regelt.

## Patentansprüche

1. Mammographiegerät mit einem höhenverstellbaren Objekttisch (2) und mit einem Sender (6) zum Erzeugen eines von der jeweiligen Höhe des Objekttisches (2) abhängigen, auf eine vor dem Objekttisch (2) stehende Patientin (1) projizierbaren optischen Signals (4).

2. Mammographiegerät nach Anspruch 1, wobei durch das projizierte optische Signal (4) die jeweilige Höhe des Objekttisches (2) auf der Patientin (1) abbildbar ist.

3. Mammographiegerät mit einem höhenverstellbaren Objekttisch (2) und mit einem Sender (6) zum Erzeugen eines von einem von der jeweiligen Höhe des Objekttisches (2) abhängigen, auf eine vor dem Objekttisch (2) stehende Patientin (1) projizierbaren und von dieser bei Koinzidenz zwischen der Höhe des Untersuchungsbereichs der Patientin (1) und der entsprechenden Einstellhöhe des Objekttisches (2) reflektierbaren optischen Signals (7).

4. Mammographiegerät nach Anspruch 3 mit einem der Patientin (1) unmittelbar unterhalb des Untersuchungsbereichs zuordbaren Reflektor (8) zur Reflexion des optischen Signals (4).

5. Mammographiegerät nach Anspruch 3 und/oder 4 mit einem auf das reflektierte optische Signal (7) ansprechenden Sensor (9), insbesondere in baulicher Einheit mit dem Mammographiegerät bzw. dem Objekttisch (2).

6. Mammographiegerät nach Anspruch 5 mit einer Steuereinheit (10) zum selbsttätigen Regeln der Höheneinstellung des Objekttisches (2) über einen Höhenantrieb (11) in Abhängigkeit des von dem Sensor (9) gemessenen, reflektierten Signals (7).

7. Mammographiegerät nach einem der Ansprüche 1 bis 6 mit dem das optische Signal (4) erzeugenden Sender (6) in baulicher Einheit mit dem Mammographiegerät bzw. dem Objekttisch (2).

8. Mammographiegerät nach einem der Ansprüche 1 bis 7 mit einem Lichtstrahl als optisches Signal (4, 7).

9. Mammographiegerät nach einem der Ansprüche 1 bis 8 mit einem Laserstrahl als optisches Signal (4, 7).

## Claims

1. Mammography device having a height-adjustable object table (2) and a transmitter (6) for generating an optical signal (4) which is dependent on the respective height of the object table (2) and can be projected onto a patient (1) positioned in front of the object table (2)

2. Mammography device according to claim 1, with it being possible to image the respective height of the object table (2) onto the patient (1) by means of the projected optical signal (4).

3. Mammography device having a height-adjustable object table (2) and a transmitter (6) for generating an optical signal (7) which is dependent on the respective height of the object table (2) and can be projected onto a patient (1) positioned in front of the object table (2) and reflected by this in the case of a coincidence between the height of the examination region of the patient (1) and the corresponding adjusting height of the object table (2).

4. Mammography device according to claim 3, having a reflector (8) immediately below the examination region which can be assigned to the patient (1) in order to reflect the optical signal (4).

5. Mammography device according to claim 3 and/or 4, having a sensor (9) responding to the reflected optical signal (7), in particular in the structural unit with the mammography device and/or object table (2).

6. Mammography device according to claim 5, having a control unit (10) for the automatic control of the height adjustment of the object table (2) by way of a height adjusting device (11) as a function of the signal (7) measured and reflected by the sensor (9).

7. Mammography device according to one of claims 1 to 6, having the transmitter (6) generating the optical signal (4) in a structural unit with the mammography device and/or the object table (2).

8. Mammography device according to one of claims 1 to 7 having a light beam as an optical signal (4, 7).

9. Mammography device according to one of claims 1 to 8 having a laser beam as an optical signal (4, 7).

## Revendications

1. Appareil de mammographie à table porte-objet réglable en hauteur (2) avec un émetteur (6) pour produire un signal optique (4) dépendant de la hauteur respective de la table porte-objet (2), projetable sur une patiente (1) se trouvant debout devant la table porte-objet (2).

2. Appareil de mammographie selon la revendication 1, dans lequel la hauteur respective de la table porte-objet peut être représentée sur la patiente (1) via le signal optique projeté (4).

3. Appareil de mammographie à table porte-objet réglable en hauteur (2) avec un émetteur (6) pour produire un signal optique (7) dépendant de la hauteur respective de la table porte-objet (2), projetable sur une patiente (1) se trouvant debout devant la table porte-objet (2) et réflexible par celle-ci en cas de coïncidence entre la hauteur de la zone d'examen de la patiente (1) et la hauteur de réglage correspondante de la table porte-objet (2).

4. Appareil de mammographie selon la revendication 3 avec un réflecteur (8) pour la réflexion du signal optique (4), pouvant être affecté à la patiente (1) immédiatement en-dessous de la zone d'examen.

5. Appareil de mammographie selon la revendication 3 et/ou 4 avec un capteur (9) répondant au signal optique réfléchi (7), en particulier en une unité de construction avec l'appareil de mammographie resp. la table porte-objet (2).

6. Appareil de mammographie selon la revendication 5 avec une unité de contrôle (10) pour le réglage automatique de la hauteur de la table porte-objet (2) via un entraînement en hauteur (11) en fonction du signal (7) réfléchi mesuré par le capteur (9).

7. Appareil de mammographie selon l'une des revendications 1 à 6 avec l'émetteur (6) produisant le signal optique (4), en une unité de construction avec l'appareil de mammographie resp. la table porte-objet (2).

8. Appareil de mammographie selon l'une des revendications 1 à 7 avec un rayon de lumière comme signal optique (4, 7).

9. Appareil de mammographie selon l'une des revendications 1 à 8 avec un rayon laser comme signal optique (4, 7).
